# EUROPÄISCHE PATENTSCHRIFT

(11) **EP 1 800 653 B1**
(45) Veröffentlichungstag und Bekanntmachung des Hinweises auf die Patenterteilung: **09.07.2008**
(21) Anmeldenummer: 06125860.4
(22) Anmeldetag: 12.12.2006
(51) Int. Cl.: A61K 8/37, A61K 8/41, A61Q 19/00

(54) **Kosmetisches, bevorzugt silikonfreies Hautpflegemittel enthaltend PPG-3 Benzyl ether myristate**
Cosmetic, esp. silicon-free composition comprising PPG-3 Benzyl ether myristate
Composition cosmétique, notamment exempte de silicone, comprenant le PPG-3 benzyl ether myristate

(30) Priorität: 21.12.2005 DE 102005062224
(43) Veröffentlichungstag der Anmeldung: 27.06.2007
(73) Patentinhaber: Coty Prestige Lancaster Group GmbH, 55116 Mainz (DE)
(72) Erfinder: Golz-Berner, Karin, 98000, Monaco (MC); Zastrow, Leonhard, 98000 Monaco (MC); Moyon, Roselyne, 06110 Le Cannet (FR)
(74) Vertreter: Walter, Wolf-Jürgen

(56) Entgegenhaltungen:
- US-B2- 6 881 776
- GAO ET AL: "A NEW MULTIFUNCTIONAL, SHINE-ENHANCING EMOLLIENT: PPG-3 BENZYL ETHER MYRISTATE" JOURNAL OF COSMETIC SCIENCE, SOCIETY OF COSMETIC CHEMISTS, NEW YORK, NY,, US, Bd. 55, Nr. SUPPL, 2004, Seiten S143-S150, XP009077503 ISSN: 1525-7886
- R.MOYON, K.GOLZ, L.ZASTROW: "The formulation of a Distinctive Skin Care Product" SÖFW-JOURNAL, Bd. 133, April 2007 (2007-04), Seiten 2-9,

## Beschreibung

Die Erfindung betrifft ein Hautpflegeprodukt, das keine Silikon-Bestandteile enthält.

Es ist bekannt, dass synthetische Polymere siliziumorganische Verbindungen, wie kettenförmige Polymethylsiloxane oder cyclische Polysiloxane wie Cyclotetra- oder Cyclopentasiloxane, die flüssige bis wachsartige Konsistenz aufweisen, seit einigen Jahren kosmetisch eingesetzt werden. Sie zeigen ein gutes Spreitvermögen, sind gut emulgierbar auch mit Wasser und sollen weder Hautatmung noch Wasserverdunstung behindern. Wegen ihres hydrophoben Charakters werden sie vor allem in Hautschutzcremes, Hautpflegeprodukten und Sonnenschutzmitteln sowie als Konditionierungsmittel in Haarprodukten eingesetzt. Sie werden insbesondere wegen ihres angenehmen, nicht fettigen Hautgefühls bevorzugt.

Trotz der guten Eigenschaften von Silikonen gibt es Vorbehalte bei Anwendern, nicht zuletzt auch wegen der in den 90er Jahren u.a. in ÖKÖ-TEST angestoßenen Diskussion von nachteiligen Wirkungen der Silikone bei Daueranwendung von Haarwäsche. Dies hat u.a. auch dazu geführt, dass Hersteller alternativer Kosmetika ihre Produkte heute mit dem Hinweis "frei von Paraffin, Mineralöl, Silikon und tierischen Stoffen" ausloben.

Das Dokument GAO ET AL: "A NEW MULTIFUNCTIONAL, SHINE-ENHANCING EMOLLIENT: PPG-3 BENZYL ETHER MYRISTATE" JOURNAL OF COSMETIC SCIENCE, SOCIETY OF COSMETIC CHEMISTS, NEW YORK, NY,, US, Bd. 55, Nr. SUPPL, 2004, Seiten S143-S150, XP009077503 ISSN: 1525-7886, offenbart Alternativen zu silikonhaltigen Produkten bei wenigstens gleichem angenehmen Hautgefühl und beschreibt in Tabelle 1 eine Zusammensetzung enthaltend PPG-3 Benzyl ether myristate und ein quaternäres Amin.

Es ist Aufgabe der vorliegenden Erfindung, ein kosmetisches Hautpflegeprodukt bereitzustellen, das bei wenigstens gleichem angenehmen Hautgefühl wie silikonhaltige Produkte ein gleiches oder besseres sensorisches Profil aufweist.

Eine weitere Aufgabe der Erfindung besteht darin, bei gleichen und besseren Sensorikeigenschaften den dafür erforderlichen Silikongehalt von etwa 10-15 Gew-% auf Null zu senken.

Erfindungsgemäß werden diese Aufgaben gelöst mit einer kosmetischen Zubereitung auf Basis von insbesondere Fettsäureestern, umfassend
4,9-5,6 % PPG-3 Benzyl Ether Myristate;
3,8-4,5 % eines Esters, abgeleitet von n-Octan- und
n-Decansäure und 2-Ethyl-hydroxymethyl-1,3-propandiol;
2,5-3,5 % eines von Milchsäure und n-Dodecylalkohol abgeleiteten Esters;
2,5-3,5 % eines Di-Esters, abgeleitet von 2,2-Dimethylpropylglycol und einem Gemisch von n-Octansäure und n-Decansäure; 1,0-2,0 % eines aus einem Gemisch von Stearinsäure und 2,2-bis (hydroxymethyl)-1,3-propandiol abgeleiteten Esters; und
1,0-2,0 % eines quarternären Amins von Rapsöl, das C₂₂-Fettsäureanteile enthält;
und einen Rest bis 100 % kosmetische Hilfsstoffe, Trägerstoffe und Gemische davon, wobei alle Prozentangaben Gewichtsprozent und auf das Gesamtgewicht der Zubereitung bezogen sind.

Zu den kosmetischen Hilfs- und Trägerstoffe, wie sie üblicherweise in solchen Zubereitungen verwendet werden, gehören z.B. Wasser, Konservierungsmittel, Farbstoffe, Pigmente mit färbender Wirkung, Verdickungsmittel, Duftstoffe, Alkohole, Gelbildner.

Zu den kosmetischen Wirkstoffen, die ebenfalls enthalten sein können, gehören z.B. anorganische und organische Lichtschutzmittel, Selbstbräunungsmittel, Radikalfänger, Feuchthaltemittel, Vitamine, Enzyme, pflanzliche Wirkstoffe, Antioxidationsmittel, entzündungswidrige natürliche Wirkstoffe, mit Sauerstoff beladene asymmetrische lamellare Aggregate gemäß WO 94/00109; Aufschlussprodukte von Hefen oder pflanzlichen Stoffen, hergestellt durch ein schonendes Ultraschall-Aufschlussverfahren gemäß WO 94/13783, Kaolin sowie mit SiO₂ modifiziertes Kaolin gemäß WO94/17588.

Für das Sensorik-Profil eines erfindungsgemäßen Hautpflegeproduktes sind solche Eigenschaften zu berücksichtigen, wie Entnahmeverhalten aus dem Behälter, Gleiten des Cremekörpers zwischen den Fingern (Textur), Spreitvermögen auf der Haut, Filmdicke beim Verteilen (Spreiten), Dauer der Verteilung, Klebrigkeit beim Verteilen, Puder- oder Seideneffekt nach dem Spreiten und nach der Filmbildung, Weichheit nach dem Spreiten, Mattierungsfinish, allgemeiner Hautkomfort.

Es wurde gefunden, dass das erfindungsgemäße Hautpflegeprodukt in der Mehrzahl solcher Sensorikeigenschaften besser abschneidet als ein ähnliches silikonhaltiges Produkt. Insbesondere sind die Textur, der allgemeine Hautkomfort und der Puder- bzw. Seideneffekt auf der Haut besser als bei einem silikonhaltigen Produkt. Das ist umso überraschender, da nicht-silikonhaltige Hautprodukte seit vielen Jahren auf dem Markt sind und die Variationsmöglichkeiten ziemlich ausgereizt sind. Außerdem sind silikonhaltige Produkte wegen ihrer weiter oben geschilderten positiven Eigenschaften oft als deutliche besserer Ersatz für traditionelle kosmetische Formeln angesehen worden.

Das erfindungsgemäße Produkt enthält auch keine Öle, wie Mineralöl oder pflanzliche Öle oder hydrierte synthetische Öle.

Als Hilfsstoffe eingesetzte Pigmente, Pigmentgemische oder Pulver mit pigmentartiger Wirkung, worunter auch solche mit Perlglanz-Effekt zu verstehen sind, können zum Beispiel umfassen Eisenoxide, natürliche Aluminiumsilicate wie Ocker, Titandioxid, Zinkoxid, Glimmer, Kaolin, manganhaltige Tone, Calciumcarbonat, Talkum, Glimmer-Titanoxid, Glimmer-Titanoxid-Eisenoxid, Wismutoxychlorid, Nylonkügelchen, Keramikkügelchen, expandierte und nichtexpandierte synthetische Polymerpulver, pulverförmige natürliche organische Verbindungen wie gemahlene Festalgen, gemahlene Pflanzenteile, verkapselte und unverkapselte Getreidestärken.

Zu Antioxidationsmitteln oder Radikalfängern gehören Vitamine wie Vitamin C und Derivate davon, beispielsweise Ascorbylacetat, -phosphat und -palmitat, Magnesiumascorbylphosphat; Vitamin A und Derivate davon; Folsäure und deren Derivate, Vitamin E und deren Derivate, wie Tocopherylacetat; Flavone oder Flavonoide; Aminosäuren, wie Histidin, Glycin, Tyrosin, Tryptophan und Derivate davon; Imidazole wie z.B. cis- oder trans-Urocaninsäure und deren Derivate; Peptide wie D,L-Carnosin, D-Carnosin, L-Carnosin und deren Derivate; Carotinoide und Carotine, wie z.B α-Carotin, β-Carotin, Lycopin; Harnsäure und Derivate davon; α-Hydroxysäuren wie Citronensäure, Milchsäure, Apfelsäure; α-Hydroxyfettsäuren wie Palmitinsäure, Phytinsäure, Lactoferrin; Stilbene und deren Derivate; Mannose und deren Derivate; Liponsäure und deren Derivate, z.B. Dihydroliponsäure; Ferulasäure und deren Derivate; Thiole wie Glutathion, Cystein, Cystin und deren Ester; Folsäure und deren Derivate.

Humectants oder Feuchthaltemittel, die enthalten sein können, sind z.B. Glycerin, Butylenglycol, Propylenglycol und Gemische davon.

Weitere Wirkstoffe sind entsprechende wasser- und/oder öllösliche UVA- oder UVB-Filter oder beide. Zu vorteilhaften öllöslichen UVB-Filtern gehören 4-Aminobenzoesäure-Derivate wie der 4-(Dimethylamino)-benzoesäure-(2-ethylhexyl)ester; Ester der Zimtsäure wie der 4-Methoxyzimtsäure(2-ethylhexyl)ester, Benzophenon-Derivate wie 2-Hydroxy-4-methoxybenzophenon; 3-Benzylidencampher-Derivate wie 3-Benzylidencampher.

Zu UVA-Filtern gehören Dibenzoylmethan-Derivate wie 1-Phenyl-4-(4'-isopropylphenyl)propan-1,3-dion, Butyl Methoxybenzoylmethane oder Menthyl Anthranilate.

Weiterhin einsetzbar als Sonnenschutzfilter sind anorganische Pigmente auf Basis von Metalloxiden, wie TiO₂, SiO₂, ZnO, Fe₂O₃, ZrO₂, MnO, Al₂O₃, die auch im Gemisch verwendet werden können.

Auch hautbräunende Wirkstoffe können in dem erfindungsgemäßen Produkt enthalten sein, z.B. Selbstbräunungsmittel wie Isatin, Alloxan, Ninhydrin, Glycerinaldehyd, meso-Weinsäurealdehyd, Glutaraldehyd, Erythrulose, Pirazolin-4,5-dionderivate, Dihydroxyacetaon (DHA), 4,4-Dihydroxypirazolin-5-dionderivate, Mahakanni-Extrakt, Glyccyhriza glabra (Süßholzwurzel), Arbutin und Gemische davon.

Ein weiterer vorteilhafter Zusatz für das erfindungsgemäße Produkt ist eine Wirkstoffzubereitung mit hohem Radikalschutzfaktor mit einem Gehalt an einem durch Extraktion der Rinde von Quebracho blanco und nachfolgender enzymatischer Hydrolyse gewonnenem Produkt, das wenigstens 90 Gew-% Proanthocyanidin-Oligomere und höchstens 10 Gew-% Gallussäure enthält, in Mikrokapseln, sowie einem durch Extraktion gewonnenen Seidenraupenextrakt, der das Peptid Cecropine, Aminosäuren und ein Vitamingemisch enthält, und einem nichtionischen, kationischen oder anionischen Hydro-Gel oder Gemisch von Hydro-Gelen, und einem oder mehreren Phospholipiden, und Wasser (z.B. gemäß WO 99/66881 Wirkstoffkomplex gemäß Beispiel 1 oder 2, oder gemäß WO 01/26617 z.B. Wirkstoffkomplex gemäß Beispiel 1 oder gemäß WO 2004/105706. Letzteres stellt ein liposomenfreies Gemisch von Pflanzenextrakten auf alkoholischer Basis dar mit einem Radikalschutzfaktor im Bereich von 1400-2900 x 10¹⁴ Radikale pro mg.

Einen weiteren vorteilhaften Wirkstoff, den das erfindungsgemäße Kosmetikum enthalten kann, sind fein verteilte hartmagnetische Einbereichsteilchen (Einkristalle) mit hoher Koerzitivfeldstärke von 3000 bis 5000 Oerstedt und mit Korngrößen im Bereich von 50 bis 1200 nm, vorzugsweise 50-250 nm, mit oder ohne die o.g. asymmetrischen lamellaren Aggregate, wobei diese hartmagnetischen Teilchen insbesondere Barium- und/oder Strontiumhexaferrite sind, erzeugt nach der Glaskristallisationstechnik durch Züchtung von Einkristallen aus einer abgeschreckten Glasschmelze (siehe WO 95/03061 z.B. Beispiel 2 oder 3; und WO 98/44895 z.B. Beispiel 1C).

Das erfindungsgemäße Produkt kann als O/W-Emulsion, W/O-Emulsion oder auch als Gel vorliegen.

Zu geeigneten Gelbildnern gehören Carbomer, Xanthangummi, Carrageenan, Akaziengummi, Guargummi, Agar-Agar, Alginate und Tylosen, Carboxymethylcellulose, Hydroxyethylcellulose, quaternisierte Cellulose, quaternisierter Guar, bestimmte Polyacrylate, Polyvinylalkohol, Polyvinylpyrrolidon, Montmorillonit.

Die Erfindung soll nachstehend durch Beispiele näher erläutert werden. Alle Angaben erfolgen in Gewichtsprozent, sofern nichts anderes angegeben ist.

### Beispiel 1 Hautpflegelotion

(Die angegebenen Bestandteile werden mehrheitlich mit den INCI-Namen bezeichnet)

| **Phase A** | |
|---|---|
| Deionisiertes Wasser | q.s. ad 100 |
| Glycerin | 3 |
| Hydroxypropyl Guar | 0,8 |
| Farblösung 0,50% in Wasser | 0,1 |

| **Phase B** | |
|---|---|
| PPG-3 Benzyl Ether Myristate | 5 |
| Trimethylolpropane Tricaprylate/Caprate | 4 |
| Lauryl Lactate | 3 |
| Neopentyl Glycol Dicaprylate/Dicaprate | 3 |
| Behentrimonium Methosulfate & Cetearyl Alcohol | 1,5 |
| Pentaerythrityl Distearate | 1,5 |

| **Phase C** | |
|---|---|
| Konservierungsmittel | 0,7 |
| Parfüm | 0,25 |

Die Bestandteile der Phase A wurden bei etwa 70 °C miteinander vermischt. Die separat bei etwa 70 °C vermischten Bestandteile der Phase B wurden mit der Phase A unter Rühren zusammengeführt und dann auf etwa 35 °C abgekühlt. Danach wurde die Phase C hinzugegeben and das Gemisch einige Minuten homogenisiert.

### Beispiel 2 Silicon-Lotion (Vergleichsbeispiel)

| **Phase A** | |
|---|---|
| Deionisiertes Wasser | q.s. ad 100 |
| Glycerin | 3 |
| Hydroxyethylacrylate/Sodium Acryloyldimethyl Taurate Copolymer | 0,25 |
| Farblösung 0,50% in Wasser | 0,10 |

| **Phase B** | |
|---|---|
| Bis-hydroxyethoxypropyl Dimethicone | 5 |
| Dimethicone & Dimethiccone Crosspolymer | 3 |
| Cyclopentasiloxane & Cyclotetrasiloxane | 2,5 |
| Glycerylstearate | 2 |
| Dimethicone & Cyclotetrasiloxane | 2 |
| Bis-PEG/PPG-16/16 Dimethicone Caprylic/Capric Triglyceride | 1,5 |
| Cetearyl Alcohol | 1 |
| Stearic Acid | 1 |
| Cyclopentasiloxane & Dimethiconol & Cyclotetetrasiloxane | 0,80 |

| **Phase C** | |
|---|---|
| Konservierungsmittel | 0,80 |
| Parfüm | 0,25 |

Die Verfahrensweise entsprach der von Beispiel 1, wobei die Temperaturen bei etwa 75 °C lagen und die Abkühltemperatur 35 °C betrug.

### Beispiel 3 Sensoriktest

Die folgenden Daten wurden von einer Probandengruppe mit 2x9 Probanden erhoben, die jeweils die Produkte nach Beispiel 1 und Beispiel 2 nach den folgenden Bewertungskriterien testeten und eine Note zwischen 1 und 9 vergaben. Die genannten Werte sind die Durchschnittswerte von allen 18 Probanden.
Produkt nach Beispiel 1 (Erfindung): A
Produkt nach Beispiel 2 (Silikon) : B

1. Entnahmeverhalten bei Entnahme aus dem Behälter (kein Ziehen von Fäden, gute Benetzung des Fingers, kein Abtropfen)
   A=7,4 B=5,8
   Erfindung um 1,6 Punkte besser
2. Gleiten des Cremekörpers zwischen den Fingern (Textur)
   A=6, 4 B=3,3
   Erfindung um 3,1 Punkte besser
3. Spreitvermögen auf der Haut
   A=5,9 B=7,0
   Erfindung um 1,1 Punkte schlechter
4. Filmdicke beim Verteilen
   A=4,1 B=2,8
   Erfindung um 1,3 Punkte besser
5. Zeit bis zur endgültigen Verteilung (playtime)
   A=4,8 B=3,0
   Erfindung um 1,8 Punkte schlechter
6. Klebrigkeit bei Verteilung
   A=3,4 B=2,7
   Erfindung um 0,7 Punkte schlechter
7. Puder-/Seideneffekt nach dem Verteilen
   A=4,9 B=4,1
   Erfindung um 0,8 Punkte besser
8. Puder-/Seideneffekt nach der Filmbildung
   A=4,8 B=3,7
   Erfindung um 1,1 Punkte besser
9. Weichheit der Haut nach dem Verteilen
   A=7,2 B=6,1
   Erfindung um 1,1 Punkte besser
10. Mattierungsfinish
   A=5,0 B=5,8
   Erfindung um 0,8 Punkte schlechter
11. Allgemeiner Hautkomfort (Bewertung 3, 5, 7, 8, 9, 10)
   A=7,4 B=6,1
   Erfindung um 1,3 Punkte besser

Daraus ergibt sich, dass 7 Eigenschaften besser bewertet wurden und 4 Eigenschaften mehrheitlich geringfügig schlechter. Das erfindungsgemäße Hautpflegeprodukt kann daher als wenigstens gleichwertig, teilweise sogar besser als ein vergleichbares Hautpflegeprodukt mit Silikon-Bestandteilen angesehen werden.

## Patentansprüche

1. Kosmetisches Hautpflegemittel auf Basis von insbesondere Fettsäureestern, **dadurch gekennzeichnet, dass** sie umfasst
4,9-5,6 % PPG-3 Benzyl Ether Myristate;
3,8-4,5 % eines Esters, abgeleitet von n-Octan- und
n-Decansäure und 2-Ethyl-hydroxymethyl-1,3-propandiol;
2,5-3,5 % eines von Milchsäure und n-Dodecylalkohol abgeleiteten Esters;
2,5-3,5 % eines Di-Esters, abgeleitet von 2,2-Dimethylpropylglycol und einem Gemisch von n-Octansäure und n-Decansäure; 1,0-2,0 % eines aus einem Gemisch von Stearinsäure und 2,2-Bis(hydroxymethyl)-1,3-propandiol abgeleiteten Esters; und
1,0-2,0 % eines quarternären Amins, insbesondere eines von Rapsöl, das C₂₂-Fettsäureanteile enthält;
und einen Rest bis 100 % kosmetische Hilfsstoffe, Trägerstoffe und Gemische davon, wobei alle Prozentangaben Gewichtsprozent und auf das Gesamtgewicht der Zubereitung bezogen sind.

2. Kosmetisches Hautpflegemittel nach Anspruch 1, **dadurch gekennzeichnet, dass** das Mittel Wirkstoffe enthält, wie anorganische und organische Lichtschutzmittel, Selbstbräunungsmittel, Radikalfänger, Feuchthaltemittel, Vitamine, Enzyme, pflanzliche Wirkstoffe, Antioxidationsmittel, entzündungswidrige natürliche Wirkstoffe.

3. Kosmetisches Hautpflegemittel nach Anspruch 1, **dadurch gekennzeichnet, dass** das Mittel wenigstens enthält PPG-3 Benzyl Ether Myristate, Trimethylolpropane Tricaprylate/Caprate, Lauryl Lactate, Neopentyl Glycol Dicaprylate/Dicaprate, Behentrimonium Methosulfate & Cetearyl Alcohol und Pentaerythrityl Distearate.

4. Kosmetisches Hautpflegemittel nach Anspruch 1, **dadurch gekennzeichnet, dass** das Mittel keine siliziumorganischen Verbindungen oder Öle enthält.

## Claims

1. A cosmetic skin care agent on the basis of fatty acid esters in particular, **characterized in that** it comprises
4.9-5.6% PPG-3 benzyl ether myristate;
3.8-4.5% of an ester derived from n-octanoic acid and n-decanoic acid and 2-ethyl-hydroxymethyl-1,3-propanediol;
2.5-3.5% of an ester derived from lactic acid and n-dodecyl alcohol;
2.5-3.5% of a diester derived from 2,2-dimethylpropyl glycol and a mixture of n-octanoic acid and n-decanoic acid;
1.0-2.0% of an ester derived from a mixture of stearic acid and 2,2-bis(hydroxymethyl)-1,3-propanediol; and
1.0-2.0% of a quaternary amine, especially one from rape oil containing shares of C₂₂ fatty acids;
and cosmetic adjuvants, vehicles and mixtures thereof as a balance to make 100%, wherein all percentages are in percent by weight and based on the overall weight of the preparation.

2. The cosmetic skin care agent according to claim 1, **characterized in that** the agent includes active substances such as inorganic and organic light stabilizers, self-tanning agents, free-radical scavengers, humectants, vitamins, enzymes, plant active substances, antioxidants, antiinflammatory natural active substances.

3. The cosmetic skin care agent according to claim 1, **characterized in that** the agent includes at least PPG-3 benzyl ether myristate, trimethylolpropane tricaprylate/caprate, lauryl lactate, neopentyl glycol dicaprylate/dicaprate, behentrimonium methosulfate & cetearyl alcohol and pentaerythrityl distearate.

4. The cosmetic skin care agent according to claim 1, **characterized in that** the agent does not include any organosilicon compounds or oils.

## Revendications

1. Agent cosmétique de soins de la peau à base notamment d'esters d'acide gras, **caractérisé en ce qu'**il comprend
4,9 - 5,6 % de PPG-3 benzyl éther myristate,
3,8 - 4,5 % d'un ester dérivé d'acide n-octanoïque et n-décanoïque et de 2-éthyl-hydroxyméthyl-1,3-propanediol,
2,5 - 3,5 % d'un ester dérivé d'acide lactique et d'alcool n-dodécylique,
2,5 - 3,5 % d'un diester dérivé de 2,2-diméthylpropylglycol et d'un mélange d'acide n-octanoïque et d'acide n-décanoïque,
1,0 - 2,0 % d'un ester dérivé d'un mélange d'acide stéarique et de 2,2-bis(hydroxyméthyl)-1,3-propanediol,
1,0 - 2,0 % d'une amine quaternaire, notamment d'une amine d'huile de colza qui contient des fractions d'acide gras en C₂₂,
et un restant jusqu'à 100 % d'agents auxiliaires cosmétiques, de vecteurs ou d'un mélange d'entre eux, moyennant quoi toutes les données en pourcentage sont exprimées en pourcentage de poids et se rapportent au poids total de la préparation.

2. Agent cosmétique de soins de la peau selon la revendication 1, **caractérisé en**
**ce que** l'agent contient des principes actifs, tels que des agents de stabilité à la lumière minéraux ou organiques, des agents d'autobronzage, des capteurs de radicaux libres, des agents hydratants, des vitamines, des enzymes, des principes actifs végétaux, des agents antioxydants, des principes actifs naturels anti-inflammatoires.

3. Agent cosmétique de soins de la peau selon la revendication 1, **caractérisé en ce que** l'agent contient au moins du PPG-3 benzyl éther myristate, du triméthylolpropane tricaprylate/caprate, du lauryl lactate, du néopentyl glycol dicaprylate/caprate, du béhentrimonium méthosulfate & alcool cétéarylique et du pentaérythrityl distéarate.

4. Agent cosmétique de soins de la peau selon la revendication 1, **caractérisé en ce que** l'agent ne contient pas de composés de silicium organique ni d'huiles.
